# EUROPEAN PATENT SPECIFICATION

(11) **EP 2 298 175 B1**
(45) Date of publication and mention of the grant of the patent: **14.06.2017**
(21) Application number: 10159592.4
(22) Date of filing: 12.04.2010
(51) Int. Cl.: A61B 8/00, A61B 8/08, A61B 8/12

(54) **Three-dimensional probe apparatus**
Sonde für dreidimensionale Messungen
Appareil de sonde tridimensionnel

(30) Priority: 22.09.2009 KR 20090089458
(43) Date of publication of application: 23.03.2011
(73) Proprietor: Medison Co., Ltd., Kangwon-do 250-875 (KR)
(72) Inventor: Hwang, Won Soon, Gyeonggi-do (KR); Hyeon, Yong Cheol, Seoul (KR); Kim, Seong Rae, Gyeonggi-do (KR)
(74) Representative: Schmid, Wolfgang

(56) References cited:
- EP-A1- 0 961 135
- WO-A1-2007/123352
- WO-A2-2006/127142
- US-A- 5 090 414
- US-A1- 2003 073 907
- US-A1- 2007 038 112
- US-A1- 2009 118 620
- US-B1- 6 719 700

## Description

### BACKGROUND OF THE INVENTION

### 1. Field of the Invention

The present invention relates to a three-dimensional probe apparatus and, more particularly, to a three-dimensional probe apparatus for an ultrasonic diagnostic apparatus that generates internal images of a patient body using ultrasound waves.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a cart-shaped main body for receiving main components thereof, a probe for transmitting and receiving ultrasound signals, a control panel having various switches and keys for inputting commands for manipulation of the apparatus, and a display unit for displaying an image of an ultrasonic diagnosis result.

The probe includes a transducer that converts electrical signals into sound signals or vice versa. The transducer includes an ultrasound wave vibrator assembly composed of a set of ultrasound wave vibrators, which sends ultrasound signals to a target to obtain an image of the target using the signals reflected from the target.

In recent years, with development of image professing techniques, an ultrasonic diagnostic apparatus has been developed to display a three-dimensional ultrasonic image. In such an ultrasonic diagnostic apparatus, the probe obtains the image of a three-dimensional region using a transducer which transmits and receives the ultrasound signals while moving along a preset locus.

In such a probe, since the transducer can move only along a restricted locus in an advancing direction of the probe, that is, a probing direction, the probe can obtain only an image in one direction if it does not change the probing direction. Therefore, there is a need to provide a probe apparatus that overcomes such a problem.

It should be noted that the above description is provided for understanding of the background of the invention and is not a description of a conventional technique well-known in the art.

The WO 2007/123352 A1 relates to an ultrasonic diagnosis apparatus and method for the urinary bladder. The ultrasonic diagnosis apparatus has a preliminary scan mode and a scan mode. The ultrasonic diagnosis apparatus first operates in the preliminary scan mode and operates in the scan mode after accurately detecting the location of the urinary bladder, thus measuring the amount of urine in the urinary bladder. When operating in the preliminary scan mode, the ultrasonic diagnosis apparatus receives pieces of ultrasonic information of n scan lines for a single plane, and acquires and displays an image for a corresponding plane using the pieces of received ultrasonic information. When operating in the scan mode, the ultrasonic diagnosis apparatus sequentially receives pieces of ultrasonic information of n scan lines for each of m planes from a transducer, and calculates the amount of urine in the urinary bladder using the pieces of received ultrasonic information. The ultrasonic diagnosis apparatus operates in the preliminary scan mode, so that the location of the urinary bladder can be quickly and accurately detected, therefore the amount of urine in the urinary bladder also can be quickly and accurately detected.

In the US 6,719,700 B1 systems and methods for localizing an ultrasound imaging transducer are provided. The ultrasound imaging transducer is operated in a first resonant mode to transmit an ultrasound imaging signal. Image data is generated based on this transmitted ultrasound imaging signal. The ultrasound imaging transducer is also operated in a second resonant mode to transmit or receive an ultrasound positioning signal. The position of the imaging transducer can then be determined based on the transmitted or received ultrasound positioning signal.

A surgical navigation system including an ultrasound catheter is disclosed in the US 2009/0118620 A1. The ultrasound catheter includes a flexible catheter housing defining a distal end, a transducer array disposed at least partially within the catheter housing, and a motor coupled with the transducer array. The motor is configured to rotate the transducer array in order to image a three-dimensional (3D) volume. The ultrasound catheter also includes at least one tracking element adapted to provide an estimate of a position and orientation of the distal end of the catheter housing. The at least one tracking element is disposed within the catheter housing and located immediately adjacent to the distal end.

In the EP 0 961 135 A1 a method and an apparatus for obtaining three-dimensional ultrasound, especially echographic images that allow the three-dimensional reconstruction of anatomic structures are incorporated. The document focuses on a rapid 3-D acquisition of dynamic and quantitative characteristics of human tissues to facilitate the diagnosis of unknown and complex pathology. An object like the heart of a human being is scanned by two-dimensional image scanning means to acquire cross-sectional images. The image scanning means are moved in predetermined increments for a predetermined number of times, while scanning said object. After the digitalization and recording of said cross-sectional images, together with their corresponding positions, said cross-sectional images are transformed into a three-dimensional data set by detecting the contours of said object within said cross-sectional images and by creating a wireframe volume model which represents the outer surface of said object in a three-dimensional manner. Such wireframe volume model is created by linking predefined contour points or 3-D coordinates of said object throughout e.g. an interpolation algorithm. For obtaining suitable wire-frame models, a full 3-D acquisition of the object is obsolete by achieving at the same time good dynamic and quantitative information of said object in a very short time.

The US 2003/0073907 A1 discloses an ultrasound cavital probe suitable for transrectal or other usage, including an ultrasound probe, having an outer housing, a pair of motors within one end of the housing, a first shaft operatively associated with one of the motors to provide longitudinal movement to the ultrasound transducer, while a second shaft operatively associated with the second motor, and which extents through the hollow interior of the first said shaft, provides for pivotal or rotary movement to the ultrasound transducer and probe, to furnish a 2-dimensional view of the surrounding anatomy, and which in combination with the movement from the first shaft, furnishes a 3-dimensional volumetric scan of the surrounding anatomy, along with facilitating the image plane movements normally obtained by a standard probe while used in a stepping device.

The US 5,090,414 discloses an intracavitary ultrasound probe comprising a rotating shaft rotatably supported inside an elongated body and adapted to swing or rotate a transducer element, an motor housed in a handle, and a stab needle guide extending in substantially parallel to the elongated body. The motor is arranged in a manner to be offset from the rotating shaft, and the ultrasound probe further comprises a mechanism for transmitting torque from the motor to the rotating shaft. Since, therefore, the handle is offset from the elongated body, a wide space for operating a stab needle is provided, thus ensuring easy operation of the stab needle. In addition, a wide space for employing a syringe is also provided. As a result of this structure, the patient's pain is reduced, and the probe is easy to rotate or tilt.

### SUMMARY OF THE INVENTION

The present invention is conceived to solve the problems of the related art as described above, and an aspect of the invention is to provide a three-dimensional probe apparatus configured to obtain images in many directions without changing a probing direction.

In accordance with one aspect of the invention, a three-dimensional probe apparatus includes: a drive unit generating power in a forward or rearward direction; a power transmission unit transmitting the power in association with the drive unit; a transducer receiving the power from the power transmission unit to move towards a plurality of locations for operation; and a location detector detecting a moving location of the transducer to allow the transducer to move towards the plurality of locations for operation.

The power transmission unit includes a power transmission member receiving the power from the drive unit; a wire member connecting the power transmission unit to the transducer; a first guide member guiding the wire member connected to the power transmission member to move in a first direction; and a second guide member disposed in a different direction from the first guide member and guiding the wire member to move in a second direction.

The location detector may include a first detection member provided to the power transmission unit to detect the moving location of the transducer.

The location detector may further include a plurality of second detection members operated in association with the first detection member and separated from each other by a distance corresponding to the plurality of locations.

The first detection member may be magnetized and each of the second detection members may include a sensor capable of sensing magnetism of the first detection member.

The drive unit may include a drive motor generating the power; a driving pulley coupled to a shaft of the drive motor; a driven pulley separated from the driving pulley; and a belt member transmitting the power from the driving pulley to the driven pulley.

The wire member may include a wire connected at one side thereof to the power transmission member and at the other side thereof to the transducer, and a pin rod provided to the wire.

The second direction is perpendicular to the first direction.

The probe apparatus may further include a base to which the transducer is rotatably coupled.

The three-dimensional probe apparatus may be an endocavity probe.

According to embodiments, the transducer may move towards a plurality of locations for operation and the location detector may detect a moving location of the transducer to define the location of the transducer so as to differently control the operation of the transducer in accordance with the moving location of the transducer, so that the probe apparatus can perform the probing operation in a plurality of directions at one position without changing the probing direction.

Further, according to the embodiments, the wire member may be guided to move in a plurality of directions including first and second directions, so that a space occupied by the drive unit and the power transmission unit is reduced, thereby reducing the overall size of the probe apparatus.

### BRIEF DESCRIPTION OF THE DRAWINGS

The above and other aspects, features and advantages of the invention will become apparent from the following description in conjunction with the accompanying drawings, in which:
- Fig. 1: is a perspective view of a three-dimensional probe apparatus in accordance with one embodiment of the present invention;
- Fig. 2: is a partially cut perspective view of the probe apparatus in accordance with the embodiment of the present invention;
- Fig. 3: is an enlarged view of part A of Fig. 2;
- Fig. 4: is an enlarged view of part B of Fig. 2;
- Fig. 5: is a cross-sectional view of Fig. 4;
- Fig. 6: is a block diagram of the three-dimensional probe apparatus of Fig. 2;
- Fig. 7: shows an operating state of part "A" of Fig. 2;
- Fig. 8: shows an operating state of part "B" of Fig. 2: and
- Fig. 9: is a cross-sectional view of Fig. 8.

### DETAILED DESCRIPTION OF THE EMBODIMENT

Exemplary embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

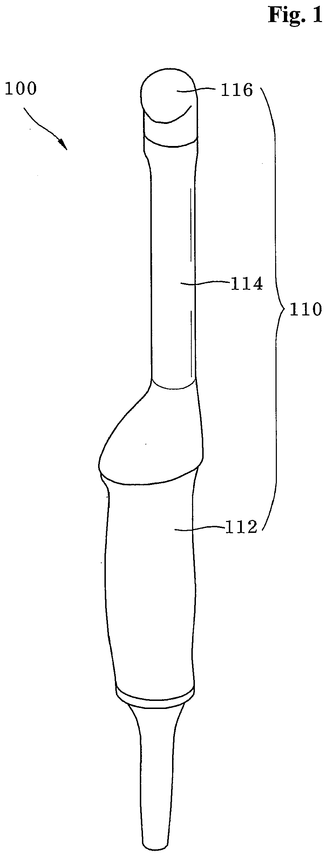

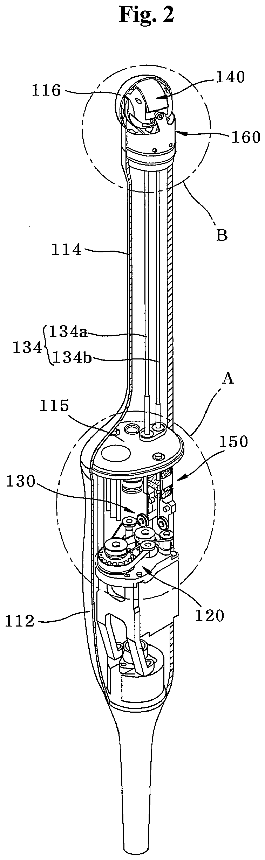

Fig. 1 is a perspective view of a three-dimensional probe apparatus in accordance with one embodiment of the present invention, and Fig. 2 is a partially cut perspective view of the probe apparatus in accordance with the embodiment of the present invention.

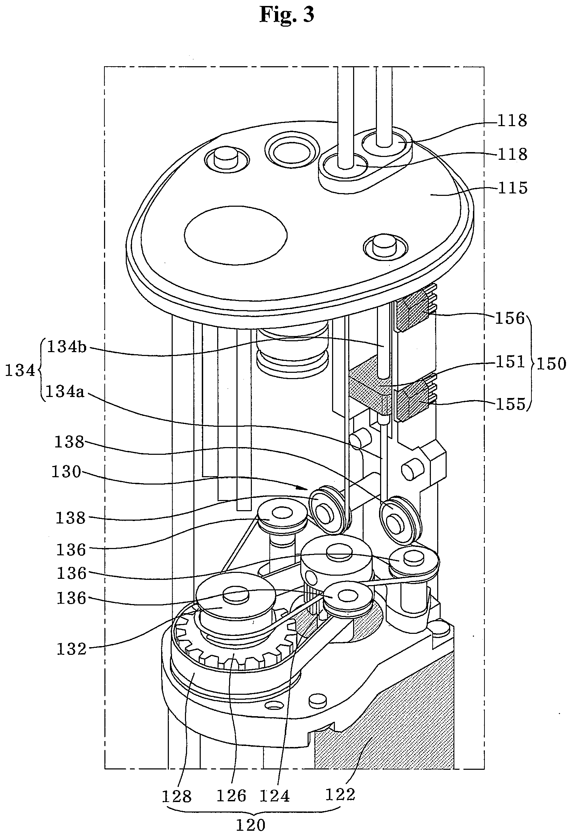

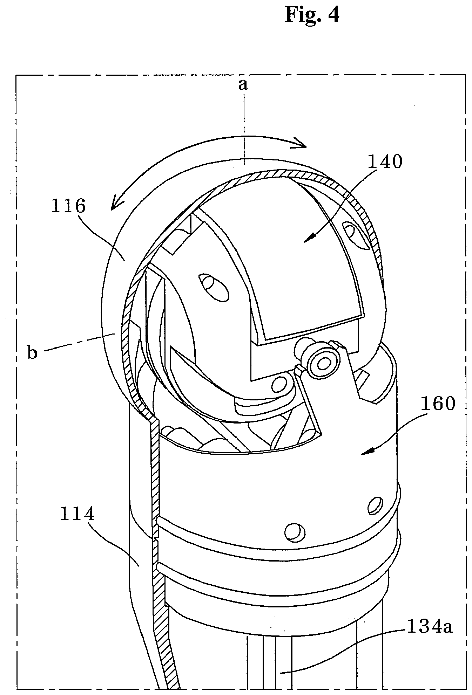

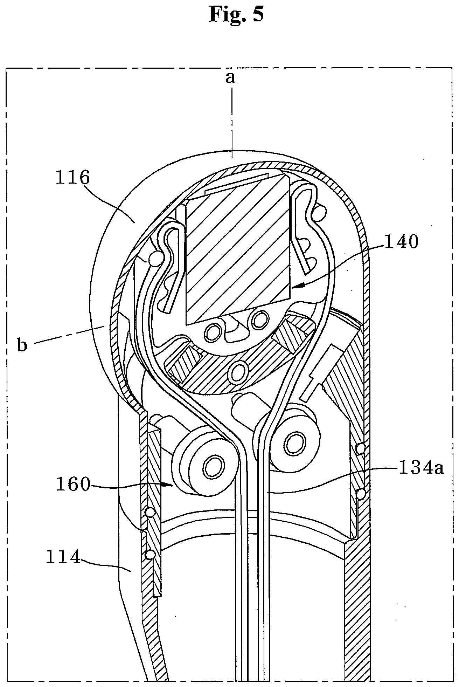

Fig. 3 is an enlarged view of part A of Fig. 2, Fig. 4 is an enlarged view of part B of Fig. 2, and Fig. 5 is a cross-sectional view of Fig. 4.

Referring to Figs 1 and 2, a three-dimensional probe apparatus 100 according to one embodiments includes a housing 110. a drive unit 120, a power transmission unit 130. a transducer 140, and a location detector 150.

The housing 110 defines an outer appearance of the probe apparatus 100 according to this embodiment. The housing 110 includes a lower housing 112, an upper housing 114, and a cover 116.

The lower housing 112 defines a lower portion of the housing 110. The lower housing 112 receives the drive unit 120 and the location detector 150.

The upper housing 114 is located on the lower housing 112 and defines an upper portion of the housing 110. The upper housing 114 receives the transducer 140 therein. Oil is also received in the upper housing 114 such that the transducer 140 is immersed in the oil inside the upper housing 114.

A partition 115 is disposed between the upper and lower housings 112 and 114. The partition 115 shields the upper and lower housings 112 and 114 from each other to prevent the oil in the upper housing 114 from leaking into the lower housing 112.

The cover 116 is disposed at an open side of the upper housing 114. The cover 116 is a component to be brought into direct contact with a diagnosis target and covers the upper housing 114 and the transducer 140.

The drive unit 120 generates power in a forward or reverse direction. The drive unit 120 is located inside the lower housing 112 and includes a drive motor 122, a driving pulley 124, a driven pulley 126, and a belt member 128, as shown in Figs. 2 and 3.

The drive motor 122 generates power. The drive motor 122 can generate a rotational force in the forward or reverse direction.

The driving pulley 124 is coupled to a shaft the drive motor 122. The driving pulley 124 receives the power from the drive motor 122 and is rotated by the rotational force of the drive motor 122 in the forward or reverse direction.

The driven pulley 126 is separated from the driving pulley 124. The driven pulley 126 is rotatably disposed and receives the power from the driving pulley 124 through the belt member 128 which transmits the power from the driving pulley 124 to the driven pulley 126.

The power transmission unit 130 transmits the power in association with the drive unit 120. The power transmission unit 130 is received inside the lower housing 112 and the upper housing 114, and includes a power transmission member 132, a wire member 134, a first guide member 136, and a second guide member 138.

The power transmission member 132 receives the power from the drive unit 120, In this embodiment, the power transmission member 132 is disposed coaxially with the driven pulley 126 and is rotated by rotation of the driven pulley 126 to receive the power from the drive unit 120. The power transmission member 132 can be rotated in the forward or reverse direction by the rotation of the driven pulley 126.

As shown in Figs. 3 to 5, the wire member 134 connects the power transmission member 132 to the transducer 140. The wire member 134 includes a wire 134a and a pin rod 134b.

The wire 134a connects the power transmission member 132 to the transducer 140. The wire 134a is connected at one side thereof with the power transmission member 132 and at the other side thereof with the transducer 140. In this embodiment, the wire 134a penetrates the partition 115 via a through-hole (reference number omitted) formed in the partition 115. The wire 134a is moved while being wound around or unwound from the power transmission member 132 by forward or rearward rotation of the power transmission member 132 to thereby move the transducer 140.

The pin rod 134b is provided to the wire 134a. The pin rod 134b is located at the middle of the wire 134a and moved together with the wire 134a. The pin rod 134b is disposed to be inserted into the through-hole in the partition 115 and has a sufficient length so as not to be deviated from the through-hole while moving together with the wire 134a.

In this embodiment, the through-hole is provided therein with an oil-seal 118 Such that the pin rod 134 is brought into close contact with the oil-seal 118 inside the through-hole. Such close contact between the pin rod 134b and the oil-seal 118 keeps the through-hole in a sealed state to thereby prevent the oil received in the upper housing 114 from leaking through the through-hole.

The first guide member 136 guides the wire member 134 connected to the power transmission member 132 to move in a first direction. The first guide member 136 is separated from the power transmission member 132 and is disposed to be rotated in the same direction as the power transmission member 132. In this embodiment, the first direction is a tangential direction to the rotating direction of the power transmission member 132.

The second guide member 138 is located in a different direction from the first guide member 136 and guides the wire member 134 to move in a second direction. The second guide member 138 is separated from the first guide member 136 and is disposed to be rotated in a direction perpendicular to the rotating direction of the first guide member 136. In this embodiment, the second direction is perpendicular to the first direction.

The wire member 134, which is moved while being wound or unwound by the rotation of the power transmission member 132, may be changed in movement direction toward the second direction by the second guide member 138 to thereby move the transducer 140.

The transducer 140 moves along a preset locus. The transducer 140 is rotatably coupled to a base 160 disposed inside the upper housing 114. While moving along the preset locus, the transducer 140 transmits an ultrasound signal to a target and receives an ultrasound echo signal reflected from the target, thereby enabling realization of a three-dimensional image.

The transducer 140 includes a piezoelectric layer (not shown) in which a piezoelectric maternal converts electrical signals into sound signals or vice versa while vibrating, a sound matching layer (not shown) reducing a difference in sound impedance between the piezoelectric layer and a target to allow as much of the ultrasound signals generated from the piezoelectric layer as possible to be transferred to the target, a lens layer (not shown) focusing the ultrasound sounds, which travel in front of the piezoelectric layer, onto a predetermined point, and a backing layer (not shown) blocking the ultrasound signals from traveling in the rearward direction of the piezoelectric layer to prevent image distortion.

The transducer 140 receives the power from the power transmission unit 130 to be moved to a plurality of locations for operation. In this embodiment, the transducer 140 may be move to a first location "a" around a second directional axis and a second location "b" around a first directional axis for operation by rotating about a rotational shaft coupled to the base 160.

In this embodiment, the transducer 140 moves right and left along a preset locus at the first location "a" and moves up and down along a preset locus at the second location "b" during operation. In order to allow such movement of the transducer 140, the wire member 134 may have a sufficient length to allow the transducer 140 to be moved not only to the first location "a" but also to the second location "b" during the operation.

The location detector 150 detects a moving location of the transducer 140 to allow the transducer 140 to be moved to the plurality of locations for operation. The location detector 150 includes a first detection member 151, and second detection members 155, 156.

The first detection member 151 is provided to the power transmission unit 130 to detect the moving location of the transducer 140. In this embodiment, the first detection member 151 is provided to the pin rod 134b of the wire member 134 and moves together with the wire member 134.

The location detector 150 includes a plurality of second detection members 155, 156, which arc operated in association with the first detection member 151. The plural second detection members 155, 156 are separated from each other by a distance corresponding to the plurality of locations. In this embodiment, the location detector 150 includes two second detection members 155, 156 which are separated from each other by a distance corresponding to the distance between the first location "a" and the second location "b". The second detection members 155, 156 detect the location of the first detection member 151 to thereby detect the moving location of the transducer 140.

In this embodiment, when the transducer 140 is located at the first location "a", the first detection member 151 is located to face the second detection member 155, which is located at a lower side of the two second detection members 155, 156. Further, when the transducer 140 is located at the second location "b", the first detection member 151 is located to face the second detection member 156, which is located at an upper side of the second detection members 155, 156.

For example, the first detection member 151 may be magnetized and the second detection members 155, 156 may be sensors capable of sensing magnetism of the first detection member 151. In this embodiment, the second detection members 155, 156 are hall sensors which detect the magnetism of the first detection member 151 facing one of the second detection members 155. 156, but not limited thereto. Alternatively, a light emitting unit may be provided as the first detection member 151 and optical sensors may be provided as the second detection members 155, 156. As such, the location detector 150 may be realized in various modifications.

The location detector 150 including the first detection member 151 and the second detection members 155, 156 detects the moving location of the transducer 140 by detecting the location of the first detection member 151 through the second detection members 155, 156.

That is, when the second detection member 155 located at the lower side detects the first detection member 151, the location detector 150 detects the moving location of the transducer 140 as the first location "a", and when the second detection member 156 located at the upper side detects the first detection member 151, the location detector 150 detects the moving location of the transducer 140 as the second location "b".

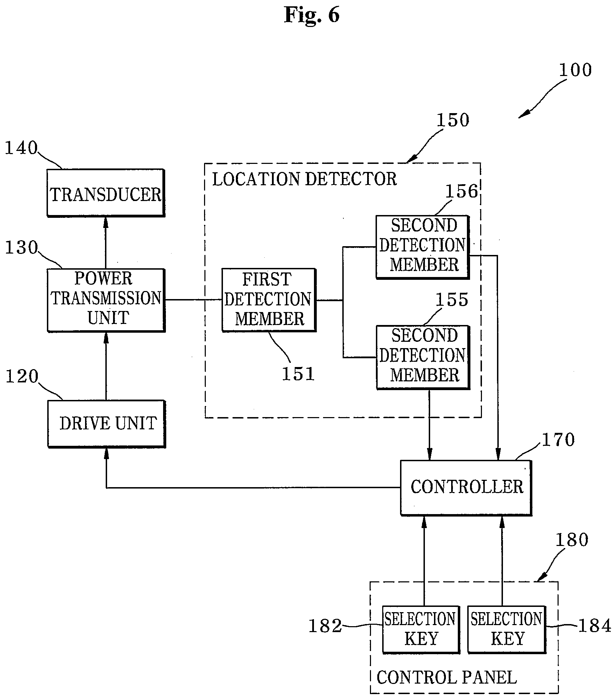

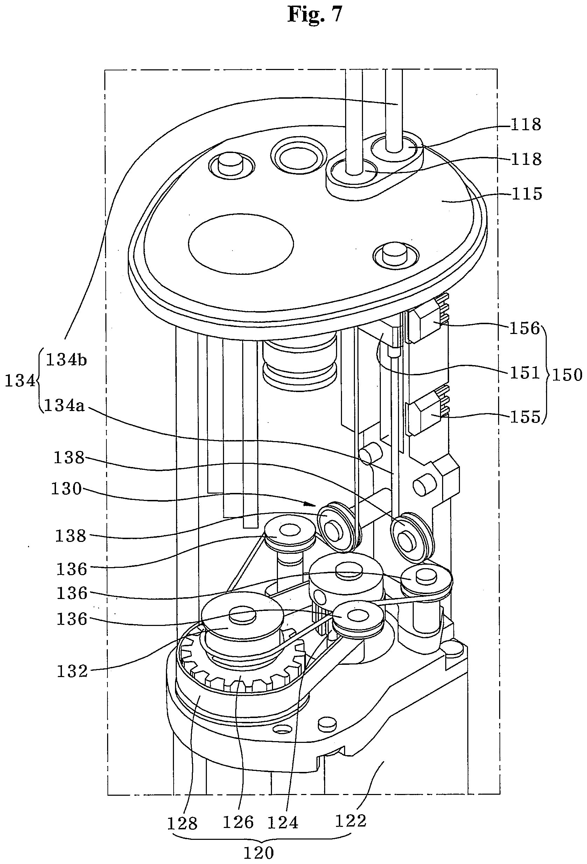

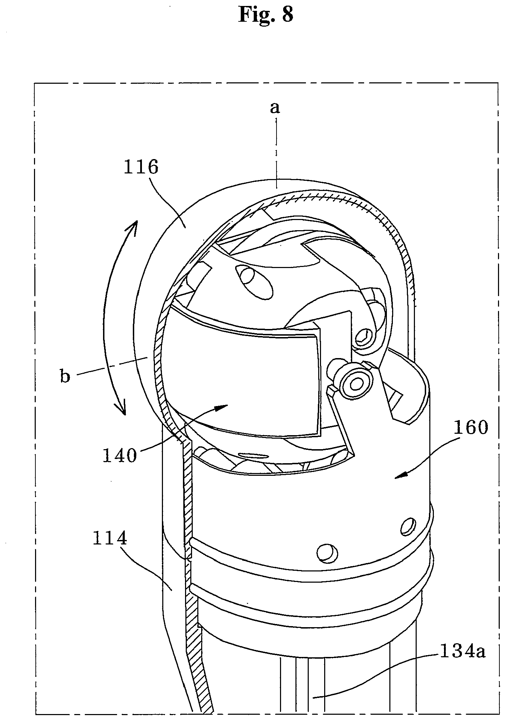

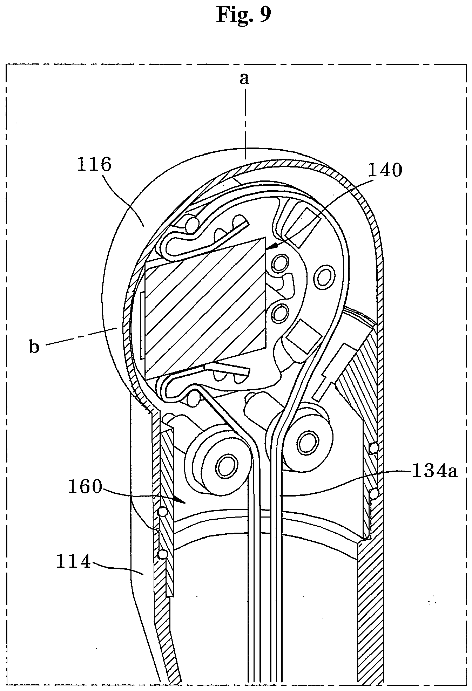

Fig. 6 is a block diagram of the three-dimensional probe apparatus of Fig. 2, Fig. 7 shows an operating state of part "A" of Fig. 2, Fig. 8 shows an operating state of part "B" of Fig. 2. and Fig. 9 is a cross-sectional view of pan "B" of Fig, 8.

Next, operation and effect of the three-dimensional probe apparatus 100 according to this embodiment will be described with reference to Figs. 3 to 9.

First, referring to Figs. 3 to 6, in the probe apparatus 100 according to this embodiment, the transducer 140 performs a probing operation with respect to a target while moving right and left along a preset locus at a first location "a", so that a three-dimensional image of the target is obtained. Such movement of the transducer 140 may be achieved by the drive unit 120 and the power transmission unit 130.

Specifically, power generated in the forward or reverse direction by the drive motor 122 of the drive unit 120 is transmitted to the power transmission member 132 of the power transmission unit 130 by connection between the driving pulley 124 and the driven pulley 126 via the belt member 128.

In this embodiment, a forward power is defined as a power in a direction of moving the transducer 140 from the first location "a" to the second location "b", and a reverse power is defined as a power in a direction of moving the transducer 140 from the second location "b" to the first location "a".

When receiving the forward or reverse power from the drive unit 120, the power transmission unit 132 transmits the forward or reverse power to the wire member 134. Then, the wire member 134 is moved in the first direction by the first guide member 136 and is moved in the second direction by the second guide members 138 while being wound around or unwound from the power transmission member 132.

While being moved in such a manner, the wire member 134 transmits the forward or reverse power of the drive unit 120 to the transducer 140 to allow the transducer 140 to move right and left along a preset locus at the first location "a".

The location of the transducer 140 is detected by the location detector 150. Namely, the first detection member 151 is moved to or near a location facing the second detection member 155, which is located at the lower side of the second detection members 155, 156, so that the second detection member 155 detects the location of the first detection member 151 to thereby detect that the transducer 140 is located at or near the first location "a".

Location information obtained by the location detector 150 is used to define the location of the transducer 140. That is, the location information of the transducer 140 obtained by the location detector 150 is transmitted to a controller 170 in a main body (not shown) of an ultrasonic diagnostic apparatus, and the controller 170 defines the location of the transducer 140 based on the location information of the transducer 140.

When the information is transmitted to the controller 170 from the location detector 150 to inform the controller 170 that the transducer 140 is located at or near the first location "a", the controller 170 controls the transducer 140 to perform the probing operation in the second direction with reference to the first position "a".

Then, the transducer 140 performs the probing operation with respect to the target while moving right and left along a preset locus with reference to the first location "a", and the controller 170 generates a three-dimensional image of the target based on an image of the target obtained by the probing operation in the second direction.

On the other hand, as shown in Figs. 6 to 9, when the transducer 140 is moved from the first location "a" to a second location "b", the transducer 140 performs the probing operation with respect to the target while moving up and down along a preset locus with reference to the first location "b", so that the three-dimensional image of the target is obtained.

Such movement of the transducer 140 to the second location "b" may be performed by user manipulation. For example, a control panel 180 of the ultrasonic diagnostic apparatus may be provided with selection keys 182, 184 for selecting whether the transducer 140 is to be moved to the first location "a" or the second location "b". When the location of the transducer 140 is selected through manipulation of the selection keys 182, 184 by a user, the controller 170 controls operation of the drive unit 120 according to the user manipulation of the selection keys 182, 184 to allow the transducer 140 to move to the first location "a" or the second location "b".

The location of the transducer 140 at the second location "b" is detected by the location detector 150. In other words, the first detection member 151 is moved to or near a location facing the second detection member 156, which is located at the upper side of the second detection members 155, 156, so that the second detection member 156 detects the location of the first detection member 151 to thereby detect that the transducer 140 is located at or near the second location "b".

Location information of the transducer 140 obtained by the location detector 150 is transmitted to the controller 170, which in turn defines the location of the transducer 140 based on the location information of the transducer 140.

When the information is transmitted to the controller 170 by the location detector 150 to inform the controller 170 that the transducer 140 is located at or near the second location "b", the controller 170 controls the transducer 140 to perform the probing operation in the second direction with reference to the second position "b".

In this embodiment, the location detector 150 includes two second detection members 155, 156 to detect movement of the transducer 140 towards two locations, but it should be noted that this invention is not limited thereto. Alternatively, the location detector 150 may include three or more second detection members to detect movement of the transducer 140 towards two or more locations. As such, it should be noted that the invention can be realized via various modifications.

In this embodiment, the three-dimensional probe apparatus 100 is an endocavity probe. Thus, when the three-dimensional probe apparatus 100 performs a probing operation after being inserted into the body cavity of a person, the probe apparatus can perform, at one location, not only a probing operation with respect to the second direction, that is, an insertion direction, by operation of the transducer 140 around the first location "a", but also a probing operation with respect to the first direction, that is, a lateral direction, by operation of the transducer 140 around the second location "b".

According to the embodiment, the transducer 140 may move towards a plurality of locations for operation and the location detector 150 may detect a moving location of the transducer 140 to define the location of the transducer 140 so as to differently control the operation of the transducer 140 in accordance with the moving location of the transducer 140, so that the probe apparatus can perform the probing operation in a plurality of directions at one location without changing the probing direction.

Further, according to the embodiment, the wire member 134 may be guided to move in a plurality of directions including first and second directions, so that a space occupied by the drive unit 120 and the power transmission unit 130 is reduced, thereby reducing the overall size of the probe apparatus.

Although some embodiments have been provided to illustrate the invention in conjunction with the drawings, it will be apparent to those skilled in the art that the embodiments arc given by way of illustration only, and that various modifications and equivalent embodiments can be made without departing from the scope of the invention. Further, the description of the three-dimensional probe apparatus as provided herein is only one example of the invention, and the invention can be applied not only to the three-dimensional probe apparatus but also to a two-dimensional probe apparatus. The scope of the invention should be limited only by the accompanying claims.

## Claims

1. A three-dimensional probe apparatus (100), comprising:
a drive unit (120) generating power in a forward or rearward direction;
a power transmission unit (130) transmitting the power in association with the drive unit (120);
a transducer (140) receiving the power from the power transmission unit (130) to move towards a plurality of locations for operation;
a location detector (150) detecting a moving location of the transducer (140) to allow the transducer (140) to move towards the plurality of locations for operation; and **characterised in that** the power transmission unit (130) further comprises:
a power transmission member (132) receiving the power from the drive unit (120);
a wire member (134) connecting the power transmission unit (130) to the transducer (140);
a first guide member (136) guiding the wire member (134) connected to the power transmission member (132) to move in a first direction; and
a second guide member (138) disposed in a different direction from the first guide member (136) and guiding the wire member (134) to move in a second direction; and
wherein the second direction is perpendicular to the first direction.

2. The three-dimensional probe apparatus (100) according to claim 1, **characterized in that** the location detector (150) comprises a first detection member (151) provided to the power transmission unit (130) to detect the moving location of the transducer (140).

3. The three-dimensional probe apparatus (100) according to claim 2, **characterized in that** the location detector (150) further comprises a plurality of second detection members (155, 156) operated in association with the first detection member (151) and separated from each other by a distance corresponding to the plurality of locations.

4. The three-dimensional probe apparatus (100) according to claim 3, **characterized in that** the first detection member (151) is magnetized and each of the second detection members (155, 156) comprises a sensor capable of sensing magnetism of the first detection member (151).

5. The three-dimensional probe apparatus (100) according to any one of claims 1 to 4, **characterized in that** the drive unit (120) comprises:
a drive motor (122) generating the power;
a driving pulley (124) coupled to a shaft of the drive motor (122);
a driven pulley (126) separated from the driving pulley (124); and
a belt member (128) transmitting the power from the driving pulley (124) to the driven pulley (126).

6. The three-dimensional probe apparatus (100) according to claim 1, **characterized in that** the wire member (134) comprises:
a wire (134a) connected at one side thereof to the power transmission member (132) and at the other side thereof to the transducer (140); and
a pin rod (134b) provided to the wire (134a).

7. The three-dimensional probe apparatus (100) according to any one of claims 1 to 4, **characterized by** further comprising:
a base (160) to which the transducer (140) is rotatably coupled.

8. The three-dimensional probe apparatus (100) according to any one of claims 1 to 4, **characterized in that** the three-dimensional probe apparatus (100) is an endocavity probe.

## Patentansprüche

1. Dreidimensionale Sondenvorrichtung (100), welche Folgendes aufweist:
eine Antriebseinheit (120), die Leistung in einer Vorwärts- oder RückwärtsRichtung erzeugt;
eine Leistungsübertragungseinheit (130), welche die Leistung in Übereinstimmung mit der Antriebseinheit (120) überträgt;
einen Transducer (140), der die Leistung von der Leistungsübertragungseinheit (130) empfängt, um sich in Richtung einer Vielzahl von Positionen für den Betrieb zu bewegen;
einen Positionsdetektor (150), der eine sich bewegende Position des Transducers (140) detektiert, um es dem Transducer (140) zu erlauben, sich in Richtung der Vielzahl von Positionen für den Betrieb zu bewegen;
und
**dadurch gekennzeichnet, dass**
die Leistungsübertragungseinheit (130) des Weiteren folgendes aufweist:
ein Leistungsübertragungselement (132), das die Leistung von der Antriebseinheit (120) empfängt;
ein Leitungselement (134), das die Leistungsübertragungseinheit (130) mit dem Transducer (140) verbindet;
ein erstes Leitelement (136), welches das Leitungselement (134), das mit dem Leistungsübertragungselement (132) verbunden ist, leitet, um sich in einer ersten Richtung zu bewegen; und
ein zweites Leitelement (138), das in einer unterschiedlichen Richtung von dem ersten Leitelement (136) angeordnet ist und das Leitungselement (134) leitet, um sich in einer zweiten Richtung zu bewegen; und
wobei die zweite Richtung senkrecht zu der ersten Richtung ist.

2. Dreidimensionale Sondenvorrichtung (100) nach Anspruch 1,
**dadurch gekennzeichnet, dass**
der Positionsdetektor (150) ein erstes Detektionselement (151) aufweist, das der Leistungsübertragungseinheit (130) zugeordnet ist, um die sich bewegende Position des Transducers (140) zu detektieren.

3. Dreidimensionale Sondenvorrichtung (100) nach Anspruch 2,
**dadurch gekennzeichnet, dass**
der Positionsdetektor (150) des Weiteren eine Vielzahl von zweiten Detektionselementen (155, 156) aufweist, die in Zusammenarbeit mit dem ersten Detektionselement (151) betrieben werden und die um einen Abstand voneinander getrennt sind, der der Vielzahl von Positionen entspricht.

4. Dreidimensionale Sondenvorrichtung (100) nach Anspruch 3,
**dadurch gekennzeichnet, dass**
das erste Detektionselement (151) magnetisiert ist und jedes der zweiten Detektionselemente (155, 156) einen Sensor aufweist, der in der Lage ist, einen Magnetismus des ersten Detektionselements (151) zu detektieren.

5. Dreidimensionale Sondenvorrichtung (100) nach einem der Ansprüche 1 bis 4,
**dadurch gekennzeichnet, dass**
die Antriebseinheit (120) folgendes aufweist:
einen Antriebsmotor (122), der die Leistung erzeugt;
eine Antriebsriemenscheibe (124), die mit einer Welle des Antriebsmotors (122) verbunden ist;
eine angetriebene Riemenscheibe (126), die von der Antriebsriemenscheibe (124) getrennt ist; und
ein Riemenelement (128), das die Leistung von der Antriebsriemenscheibe (124) zu der angetriebenen Riemenscheibe (126) überträgt.

6. Dreidimensionale Sondenvorrichtung (100) nach Anspruch 1,
**dadurch gekennzeichnet, dass**
das Leitungselement (134) Folgendes aufweist:
eine Leitung (134a), die an einer Seite derselben mit dem Leistungsübertragungselement (132) und an der anderen Seite desselben mit dem Transducer (140) verbunden ist; und
einen Anschlussstab (134b), der der Leitung (134a) zugeordnet ist.

7. Dreidimensionale Sondenvorrichtung (100) nach einem der Ansprüche 1 bis 4,
**dadurch gekennzeichnet, dass**
sie des Weiteren folgendes aufweist:
einen Grundkörper (160), mit dem der Transducer (140) drehbar verbunden ist.

8. Dreidimensionale Sondenvorrichtung (100) nach einem der Ansprüche 1 bis 4
**dadurch gekennzeichnet, dass**
die dreidimensionale Sondenvorrichtung (100) eine Endokavitär-Sonde ist

## Revendications

1. Appareil de sonde tridimensionnelle (100), comprenant :
une unité d'entraînement (120) générant de la puissance dans une direction avant ou une direction arrière ;
une unité de transmission de puissance (130) pour transmettre de la puissance en association avec l'unité d'entraînement (120) ;
un transducteur (140) recevant la puissance de l'unité de transmission de puissance (130) pour assurer le déplacement vers une pluralité de localisations pour opérer ;
un détecteur de localisation (150) détectant une localisation de mouvement du transducteur (140) pour permettre au transducteur (140) de se déplacer vers la pluralité de localisations pour opérer ; et
**caractérisé en ce que** l'unité de transmission de puissance (130) comporte en outre :
un élément de transmission de la puissance (132) recevant la puissance de l'unité d'entraînement (120) ;
un élément de raccordement câblé (134) connectant l'unité de transmission de puissance (130) au transducteur (140) ;
un premier élément de guidage (136) qui guide l'élément de raccordement câblé (134) connecté à l'élément de transmission de la puissance (132) pour assurer le déplacement dans une première direction ; et
un second élément de guidage (138), disposé dans une direction différente de celle du premier élément de guidage (136) et qui guide l'élément de raccordement câblé (134) pour se déplacer dans une seconde direction ;
et
dans lequel la seconde direction est perpendiculaire à la première direction.

2. Appareil de sonde tridimensionnelle (100) selon la revendication 1, **caractérisé en ce que** le détecteur de localisation (150) comprend un premier organe de détection (151) affecté à l'unité de transmission de puissance (130) agencé pour détecter la localisation du mouvement du transducteur (140).

3. Appareil de sonde tridimensionnelle (100) selon la revendication 2, **caractérisé en ce que** le détecteur de localisation (150) comprend en outre une pluralité de seconds éléments de détection (155, 156) opérant en association avec le premier élément de détection (151) et séparés les uns des autres par une distance correspondant à la pluralité de localisations.

4. Appareil de sonde tridimensionnelle (100) selon la revendication 3, **caractérisé en ce que** le premier élément de détection (151) est magnétisé et chacun des seconds éléments de détection (155, 156) comprend un capteur capable de détecter le magnétisme du premier élément de détection (151).

5. Appareil de sonde tridimensionnelle (100) selon l'une quelconque des revendications 1 à 4, **caractérisé en ce que** l'unité d'entraînement (120) comprend :
un moteur d'entraînement (122) qui génère la puissance ;
une poulie d'entraînement (124) couplée à un arbre du moteur d'entraînement (122) ;
une poulie entraînée (126) séparée de la poulie d'entraînement (124) ; et
une courroie (128) transmettant la puissance de la poulie d'entraînement (124) à la poulie entraînée (126).

6. Appareil de sonde tridimensionnelle (100) selon la revendication 1, **caractérisé en ce que** l'élément de raccordement câblé (134) comprend :
un câble (134a) connecté à un côté de l'élément de transmission de la puissance (132) et de l'autre côté au transducteur (140) ; et
une goupille (134b) attachée au câble (134a).

7. Appareil de sonde tridimensionnelle (100) selon l'une quelconque des revendications 1 à 4, **caractérisé en ce qu'**il comprend en outre :
une embase (160) sur lequel le transducteur (140) est monté en rotation.

8. Appareil de sonde tridimensionnelle (100) selon l'une quelconque des revendications 1 à 4, **caractérisé en ce que** l'appareil de sonde tridimensionnelle (100) est une sonde d'endocavité.
